# EUROPÄISCHE PATENTSCHRIFT

(11) **EP 1 319 645 B1**
(45) Veröffentlichungstag und Bekanntmachung des Hinweises auf die Patenterteilung: **26.10.2005**
(21) Anmeldenummer: 02027113.6
(22) Anmeldetag: 04.12.2002
(51) Int. Cl.: C07C 29/80, C07C 31/137, C07C 35/37, C07C 29/82, C07C 29/84

(54) **Verfahren zur Reinigung alicyclischer Alkohole**
Process for the purification of alicyclic alcohols
Procédé pour la purification d'alcools alicycliques

(30) Priorität: 14.12.2001 DE 10161597
(43) Veröffentlichungstag der Anmeldung: 18.06.2003
(73) Patentinhaber: Celanese Chemicals Europe GmbH, 60439 Frankfurt am Main (DE)
(72) Erfinder: Dukat, Wolfgang, Dr., 46147 Oberhausen (DE); Lappe, Peter, Dr., 46539 Dinslaken (DE); Schmid, Klaus, Dr., 46539 Dinslaken (DE); Scholz, Horst, 46535 Dinslaken (DE); Storm, Edgar, 46147 Oberhausen (DE)

(56) Entgegenhaltungen:
- EP-A- 0 378 756
- WO-A-96/26173
- DE-A- 4 226 282
- US-A- 2 889 375
- US-A- 3 990 952
- US-A- 5 264 638
- DATABASE WPI Section Ch, Week 200169 Derwent Publications Ltd., London, GB; Class A41, AN 2001-604893 XP002253295 & JP 2001 131105 A (DAICEL CHEM IND LTD), 15. Mai 2001 (2001-05-15)

## Beschreibung

Die vorliegende Erfindung betrifft ein Verfahren zur destillativen Reinigung alicyclischer Alkohole in Gegenwart sauer reagierender Verbindungen.

Alicyclische Alkohole basieren auf ein- oder mehrcyclischen aliphatischen Kohlenwasserstoffen, die eine oder mehrere Hydroxylgruppen tragen.

Alicyclische Alkohole besitzen eine hohe wirtschaftliche Bedeutung. Sie finden eine breite Anwendung bei der Synthese von Alkydharzen, Polyestern, Polyacrylaten oder Polycarbonaten für Lacke, Farben oder Klebstoffe, wie zum Beispiel aus der deutschen Offenlegungsschrift DE-OS-1 916 287 bekannt.

Die Herstellung dieser alicyclischen Alkohole erfolgt bevorzugt durch Hydroformylierung von mono- oder mehrcyclischen Olefinen mit nachfolgender Hydrierung der intermediär gebildeten Aldehyde. Eine zusammenfassende Darstellung findet sich beispielsweise in J. Falbe, Synthesen mit Kohlenmonoxid, Springer Verlag Berlin 1967, S.34 ff, Cornils, Chemiker Zeitung, 98. Jahrgang, 1974, Seite 70 ff oder in J. Falbe und N. Huppes, Brennstoffchemie 48 (1967), 182.

Bedeutende alicyclische Alkohole leiten sich vom Dicyclopentadien (Tricyclo[5.2.1.0^{2,6}]deca-3,8-dien) ab. Die Hydroformylierung dieses Ausgangsolefins und die nachfolgende Hydrierung gestattet einen Zugang zu den sogenannten Tricyclodecan-Alkoholen, die im Stand der Technik häufig auch als TCD-Alkohole bezeichnet werden. Solche TCD-Alkohole sind beispielsweise TCD-Alkohol DM (Isomerengemisch enthaltend 3(4),8(9)-Bis(hydroxymethyl)tricyclo[5.2.1.0^{2,6}]decan) oder TCD-Alkohol OM (Isomerengemisch enthaltend 8(9)-Hydroxy-3(4)-hydroxymethyltricyclo[5.2.1.0^{2,6}]decan), der durch Hydroformylierung von TCD-Alkohol E (8(9)-Hydroxy-tricyclo[5.2.1.0^{2,6}]dec-3-en) nach der DE-AS-1 018 415 zugänglich ist. Bei der Bezeichnung des Isomerengemisches als 3(4),8(9)-Bis(hydroxymethyl)tricyclo[5.2.1.0^{2,6}]decan befindet sich ein am Tricyclodecangerüst gebundener Hydroxymethylrest in der 3- oder 4-Position und der andere Hydroxymethylrest in der 8- oder 9-Position.

Dementsprechend steht die Bezeichnung 3(4),8(9)-Bis(hydroxymethyl)tricyclo[5.2.1.0^{2,6}]decan für ein Gemisch enthaltend 3,8-Bis(hydroxymethyl)tricyclo[5.2.1.0^{2,6}]decan, 3,9-Bis(hydroxymethyl)tricyclo[5.2.1.0^{2,6}]decan, 4,8-Bis(hydroxymethyl)tricyclo[5.2.1.0^{2,6}]decan und 4,9-Bis(hydroxymethyl)tricyclo[5.2.1.0^{2,6}]decan und die Bezeichnung 8(9)-Hydroxy-3(4)-hydroxymethyltricyclo[5.2.1.0^{2,6}]decan für ein Gemisch enthaltend 8-Hydroxy-3-hydroxymethyltricyclo[5.2.1.0^{2.6}]decan, 9-Hydroxy-3-hydroxymethyltricyclo[5.2.1.0^{2,6}]decan, 8-Hydroxy-4-hydroxymethyltricyclo[5.2.1.0^{2,6}]decan und 9-Hydroxy-4-hydroxymethyltricyclo[5.2.1.0^{2,6}]decan.

Weitere alicyclische Alkohole erhält man durch die Rhodium katalysierte Hydroformylierungsreaktion von Tricyclopentadien (Isomerengemisch enthaltend 3a,4,4a,5,8,8a,9,9a-Octahydro-4,9:5,8-dimethanobenz[f]inden und 1,4,4a,4b,5,8,8a,9a-Octahydro-1,4:5,8-dimethanofluoren) gemäß der EP-A1-1 065194 und anschließender Hydrierung zu den Alkoholen. Dabei bildet sich PCPD-Alkohol DM, bei dem es sich um ein Isomerengemisch enthaltend 1,4:5,8-Dimethano-2(3),6(7)-bis(hydroxymethyl)perhydrofluoren und 4,9:5,8-Dimethano-1(2),6(7)-bis(hydroxymethyl)perhydrobenz[f]inden handelt.

Dabei steht die Bezeichnung 1,4:5,8-Dimethano-2(3), 6(7)-bis(hydroxymethyl)perhydrofluoren für ein Gemisch enthaltend 1,4:5,8-Dimethano-2,6-bis(hydroxymethyl)perhydrofluoren, 1,4:5,8-Dimethano-2,7-bis(hydroxymethyl)perhydrofluoren, 1,4:5,8-Dimethano-3,6-bis(hydroxymethyl)perhydrofluoren und 1,4:5,8-Dimethano-3,7-bis(hydroxymethyl)perhydrofluoren sowie die Bezeichnung 4,9:5,8-Dimethano-1(2), 6(7)-bis(hydroxymethyl)perhydrobenz[f]inden für ein Gemisch enthaltend 4,9:5,8-Dimethano-1,6-bis(hydroxymethyl)perhydrobenz[f]inden, 4,9:5,8-Dimethano-2,6-bis(hydroxymethyl)perhydrobenz[f]inden, 4,9:5,8-Dimethano-1,7-bis(hydroxymethyl)perhydrobenz[f]inden und 4,9:5,8-Dimethano-2,7-bis(hydroxymethyl)perhydrobenz[f]inden.

Ein weiterer bedeutender alicyclischer Alkohol ist BCH-Alkohol M, ein Isomerengemisch enthaltend 2-Hydroxymethyl-bicyclo[2.2.1]heptan.

Für viele Anwendungsbereiche, beispielsweise bei der Herstellung von Acrylsäureestern, ist der Einsatz hochreiner Alkohole unbedingt erforderlich, um Beeinträchtigungen auf den Reaktionsverlauf von Veresterungs- oder Polykondensationsreaktionen möglichst gering zu halten. Auch ist der Einsatz hochreiner Alkohole erforderlich, um die Bildung unerwünscht gefärbter Produkte zu vermeiden. So stören insbesondere Aldehydgruppen enthaltende Verbindungen als Verunreinigungen.

Bei der technischen Herstellung der alicyclischen Alkohole über die Hydroformylierungsreaktion der entsprechenden Olefine und anschließender Hydrierung enthalten die gewünschten alicyclischen Alkohole stets Spuren an nicht vollständig hydrierten Verbindungen mit einer oder mehreren Aldehydgruppen. Beispielsweise enthält technisch verfügbarer TCD-Alkohol DM geringe Mengen an TCD-Hydroxyaldehyd oder TCD-Dialdehyd. Die Bestimmung der Aldehydgruppen enthaltenden Verunreinigungen erfolgt im allgemeinen nach DIN 53173, Ausgabe 1983-02. Ihr Gehalt wird als Carbonylzahl, auch als CO-Zahl bezeichnet, in mg KOH/g angegeben. Bei technischem TCD-Alkohol-DM liegt die Carbonylzahl im allgemeinen in einem Bereich von 0,2-0,8 mg KOH/g entsprechend einem Anteil von TCD-Hydroxyaldehyd von 0,07-0,28 Gew.-%.

Die Aldehydgruppen enthaltenden Verbindungen zeigen häufig ein den vollständig hydrierten Alkoholen ähnliches Siedeverhalten, so daß eine einfache einstufige Destillation nicht zu hochreinen alicyclischen Alkoholen führt. Bisher erfolgt die Reinigung durchwegs über eine mehrstufige fraktionierte Destillation mit einer hohen Trennleistung. Die alicyclischen Alkohole werden hierbei einen Zeitraum von mehreren Stunden einer thermischen Belastung ausgesetzt, wobei in der Regel Sumpftemperaturen von 100 bis 240°C angewandt werden. Die Erhöhung des Destillationsaufwandes führt zum einen zu erheblichen Produktverlusten durch den Anfall von Vor- und Zwischenfraktionen, sowie der Bildung von Höhersiedern. Zum anderen ist infolge der thermischen Belastung der Rohalkohole auch eine gewisse Aldehydbildung sogar während der Destillation der Rohalkohole zu befürchten.

Es bestand daher die Aufgabe, ein Verfahren zur Destillation von alicyclischen Alkoholen bereitzustellen, das auf einfache und kostengünstige Weise die Abtrennung Aldehydgruppen enthaltender Verunreinigungen ermöglicht und auch die Bildung solcher Verunreinigungen während der thermischen Belastung bei der Destillation unterdrückt.

Aus dem Stand der Technik sind destillative Reinigungsverfahren von Oxoalkoholen unter Zusatz basischer Verbindungen bekannt. Nach dem aus US-A-2,889,375 offenbarten Verfahren werden aliphatischen, acyclischen Oxoalkoholen, die geringe Mengen an Aldehyden enthalten, im Kolonnensumpf Erdalkalimetallverbindungen, insbesondere Oxide, Hydroxide oder Carbonate in einer Menge von 1 Gew.-%, bezogen auf die Einsatzmenge, zugesetzt. Nach der Lehre der EP-B1-0 869 936 lassen sich aliphatische Alkohole in Gegenwart geringer Mengen Alkalihydroxid destillativ reinigen.

Es wurde nun überraschenderweise gefunden, dass durch die Zugabe von geringen Mengen sauer reagierender Verbindungen während der Destillation von alicyclischen Alkoholen noch vorhandene Aldehydreste beseitigt werden und die Bildung entsprechender Aldehyde unterdrückt wird.

Gegenstand der vorliegenden Erfindung ist daher ein Verfahren zur destillativen Reinigung von alicyclischen Alkoholen. Es ist dadurch gekennzeichnet, dass man die alicyclischen Alkohole in Gegenwart von 1 bis 500 ppm sauer reagierender Verbindungen, bezogen auf die Menge des eingesetzten alicyclischen Alkohols, destilliert.

Arbeitet man nach dem erfindungsgemäßen Verfahren, so genügt eine einfache Destillationsbrücke mit nur einem theoretischen Boden, um die gewünschten alicyclischen Alkohole mit einer sehr hohen Reinheit zu gewinnen. Im allgemeinen liegen die Carbonyl-Zahlen der erhaltenen Destillate bei kleiner 0,05 mg KOH/g. Da im Gegensatz zum Stand der Technik keine aufwendige Kolonnenschaltung mit einer Vielzahl von theoretischen Böden erforderlich ist sondern überraschenderweise nur eine einfache Destillationsbrücke mit nur einem theoretischen Boden genügt, erhält man vorteilhafterweise hohe destillative Ausbringungen an den gewünschten reinen Alkoholen und man muß nur geringe Destillationsverluste in Kauf nehmen. Im allgemeinen liegt die destillative Ausbringung bei über 98 % vom Einsatzprodukt.

Bei den alicyclischen Alkoholen handelt es sich um ein oder mehrwertige Alkohole, die auf ein- oder mehrcyclischen aliphatischen Kohlenwasserstoffen basieren und die im allgemeinen 7 bis 17 Kohlenstoffatome im Molekül enthalten. Alicyclische Alkohole, bei denen das erfindungsgemäße Verfahren mit besonderem Erfolg angewandt werden kann, sind TCD-Alkohol DM und TCD-Alkohol OM.

Die sauer reagierenden Verbindungen, die gemäß dem erfindungsgemäßen Verfahren zugesetzt werden können, unterliegen keiner Einschränkung und es können alle dem Fachmann geläufigen sauren Verbindungen zugesetzt werden, sofern die zugesetzten sauren Verbindungen einen ausreichend hohen Siedepunkt oder Sublimationspunkt aufweisen, damit sichergestellt wird, dass keine sauren Verbindungen in das Destillat gelangen. Vorzugsweise werden solche sauer reagierende Verbindungen eingesetzt, die bei Raumtemperatur fest sind. Unter den sauren Verbindungen eignen sich bevorzugt aliphatische oder aromatische Sulfonsäuren, die eine oder mehrere Sulfonsäuregruppen tragen. Geeignete Sulfonsäuren sind beispielsweise para-Toluolsulfonsäure, Naphthalinsulfonsäure, Sulfanilsäure oder Camphersulfonsäure. Auch die sauren Salze starker oder schwacher Mineralsäuren, wie zum Beispiel Kaliumhydrogensulfat, Natriumdihydrogenphosphat, die starken oder schwachen Mineralsäuren selbst, wie Schwefelsäure oder Phosphorsäure sind geeignet, ebenso Tetraalkylammoniumsalze starker und schwacher Mineralsäuren, wie Tetrabutylammoniumhydrogensulfat. Ebenso lassen sich auch feste Nichtmetalloxide, wie Diphosphorpentoxid oder in die acide Form überführte lonenaustauscher mit Erfolg einsetzten. Auch hochsiedende aliphatische oder aromatische ein oder mehrwertige Carbonsäuren, wie beispielsweise 9-Anthracencarbonsäure, Thapsiasäure oder Trimellithsäure, sind für den Einsatz in dem erfindungsgemäßen Verfahren geeignet, obwohl der Verwendung von Sulfonsäuren der Vorzug zu geben ist. Besonders bewährt hat es sich, para-Toluolsulfonsäure, Camphersulfonsäure oder Kaliumhydrogensulfat einzusetzen.

Die Menge an zugesetzter sauer reagierender Verbindung liegt im Bereich von 1-500 ppm, vorzugsweise im Bereich von 5-250 ppm und besonders bevorzugt im Bereich von 5-50 ppm, jeweils bezogen auf die Menge des eingesetzten alicyclischen Alkohols. Zusätze in geringeren Mengen zeigen keine ausreichende Wirkung mehr. Zusätze in höheren Mengen sind ebenfalls nicht zu empfehlen, da sie das Verfahren unnötig verteuern, die Fremdstoffbelastung erhöhen und höhere Zusätze zu Nebenreaktionen, wie der Ether- und Esterbildung, führen können. Zweckmäßigerweise werden die sauer reagierenden Verbindungen in reiner Form als Flüssigkeit oder als Feststoff dem Rohalkohol zugesetzt, vorzugsweise als Feststoff.

Für die destillative Reinigung genügt es schon, eine einfache Destillationsbrücke mit nur einer theoretischen Trennstufe einzusetzen. Die Verwendung von Destillationskolonnen ist aber nicht ausgeschlossen, obgleich bei ihrer Verwendung man dann mit höheren Destillationsverlusten zu rechnen hat. Arbeitet man mit Destillationskolonnen, so haben diese im allgemeinen 2 bis 40 Böden. Die jeweiligen Druck- und Temperaturbedingungen sind den individuell zu reinigenden Alkoholen anzupassen.

Wie die folgenden Beispiele zeigen, lassen sich bei der destillativen Aufarbeitung an einer einfachen Destillationsbrücke in Gegenwart geringer Mengen an sauer reagierenden Verbindungen alicyclische Alkohole in einer solchen Qualität erhalten, bei denen die Carbonylzahl im allgemeinen auf unter 10 %, häufig sogar auf unter 5 % des Ausgangswertes in dem Einsatzalkohol gesenkt werden kann. Da eine einfache Destillationsbrücke mit nur einem theoretischem Boden für die Trennung ausreicht, werden bei dem erfindungsgemäßen Verfahren nur geringe Destillationsverluste beobachtet.

Die folgenden Beispiele sollen die Erfindung erläutern, ohne sie auf bestimmte Ausführungsformen einzuschränken.

### Vergleichsbeispiel 1

In einem 4I-Rundkolben, bestückt mit Thermometer und Claisenbrücke, wurden 1860,1 g TCD-Alkohol DM (Carbonylzahl 0,57 mg KOH/g) vorgelegt und unter Rühren bei einer Temperatur von 80°C über einen Zeitraum von 30 Minuten mit Stickstoff durchspült. Bei einer Kopftemperatur von 172-176°C und einem Druck von 2 mbar wurden 1796,0 g TCD-Alkohol DM mit einer Carbonylzahl von 0,54 mg KOH/g erhalten. Dies entsprach einer destillativen Ausbringung von 96,6%. Die Carbonyl-Zahl wurde hierbei nur unwesentlich auf 94,0% des Ausgangswertes reduziert.

### Vergleichsbeispiel 2

In einem 4I-Rundkolben, bestückt mit Thermometer und einer Füllkörperkolonne mit 4,5 theoretischen Trennstufen wurden 1604,8 g TCD Alkohol DM (Carbonylzahl 0,22 mg KOH/g) vorgelegt und unter Rühren bei einer Temperatur von 80°C über einen Zeitraum von 30 Minuten mit Stickstoff durchspült. Nach einem Vorlauf von 410,9 g (25,6% vom Einsatz) wurden bei einer Kopftemperatur von 175°C und einem Druck von 2 mbar 1139,6 g TCD-Alkohol DM mit einer Carbonylzahl von 0,041 mg KOH/g erhalten. Dies entsprach einer destillativen Ausbringung von 71,0%. Die Carbonyl-Zahl wurde auf 18,5% des ursprünglichen Wertes verringert.

### Beispiel 1

In einem 4I-Rundkolben, bestückt mit Thermometer und Claisenbrücke, wurden 1923,4 g TCD-Alkohol DM (Carbonylzahl 0,57 mg KOH/g) vorgelegt, mit 19,2 mg para-Toluolsulfonsäure versetzt und unter Rühren bei einer Temperatur von 80°C über einen Zeitraum von 30 Minuten mit Stickstoff durchspült. Bei einer Kopftemperatur von 172-176°C und einem Druck von 2 mbar wurden 1908,4 g TCD-Alkohol DM mit einer Carbonylzahl von 0,036 mg KOH/g erhalten. Dies entsprach einer destillativen Ausbringung von 99,2%. Die Carbonylzahl wurde auf 6,4% des ursprünglichen Wertes verringert.

### Beispiel 2

Gemäß Beispiel 1 wurden 1964,9 g TCD-Alkohol DM (Carbonylzahl 0,57 mg KOH/g) mit 98,3 mg para-Toluolsulfonsäure versetzt. Bei der folgenden Destillation wurden 1928,4 g TCD-Alkohol DM mit einer Carbonylzahl von 0,038 mg KOH/g erhalten. Dies entsprach einer destillativen Ausbringung von 98,1%. Die Carbonylzahl wurde auf 6,6% des ursprünglichen Wertes verringert.

### Beispiel 3

Gemäß Beispiel 1 wurden 1938,0 g TCD-Alkohol DM (Carbonylzahl 0,57 mg KOH/g) mit 193,8 mg para-Toluolsulfonsäure versetzt. Bei der folgenden Destillation wurden 1897,2 g TCD-Alkohol DM mit einer Carbonylzahl von 0,065 mg KOH/g erhalten. Dies entsprach einer destillativen Ausbringung von 97,9%. Die Carbonylzahl wurde auf 11,4% des ursprünglichen Wertes verringert.

### Beispiel 4

Gemäß Beispiel 1 wurden 1803,5 g TCD-Alkohol DM (Carbonylzahl 0,81 mg KOH/g) mit 9,0 mg para-Toluolsulfonsäure versetzt. Bei der folgenden Destillation wurden 1775,5 g TCD-Alkohol DM mit einer Carbonylzahl von 0,053 mg KOH/g erhalten. Dies entsprach einer destillativen Ausbringung von 98,5%. Die Carbonylzahl wurde auf 6,6% des ursprünglichen Wertes verringert.

### Beispiel 5

Gemäß Beispiel 1 wurden 1866,0 g TCD-Alkohol DM (Carbonylzahl 0,81 mg KOH/g) mit 18,7 mg para-Toluolsulfonsäure versetzt. Bei der folgenden Destillation wurden 1853,3 g TCD-Alkohol DM mit einer Carbonylzahl von 0,050 mg KOH/g erhalten. Dies entsprach einer destillativen Ausbringung von 99,3%. Die Carbonylzahl wurde auf 6,2% des ursprünglichen Wertes verringert.

### Beispiel 6

Gemäß Beispiel 1 wurden 1967,5 g TCD-Alkohol DM (Carbonylzahl 0,81 mg KOH/g) mit 196,8 mg para-Toluolsulfonsäure versetzt. Bei der folgenden Destillation wurden 1928,7 g TCD-Alkohol DM mit einer Carbonylzahl von 0,032 mg KOH/g erhalten. Dies entsprach einer destillativen Ausbringung von 98,0%. Die Carbonylzahl wurde auf 4,0% des ursprünglichen Wertes verringert.

### Beispiel 7

Gemäß Beispiel 1 wurden 1936,5 g TCD-Alkohol DM (Carbonylzahl 0,57 mg KOH/g) mit 19,4 mg Camphersulfonsäure versetzt. Bei der folgenden Destillation wurden 1918,5 g TCD-Alkohol DM mit einer Carbonylzahl von 0,045 mg KOH/g erhalten. Dies entsprach einer destillativen Ausbringung von 99,1 %. Die Carbonylzahl wurde auf 7,8% des ursprünglichen Wertes verringert.

### Beispiel 8

Gemäß Beispiel 1 wurden 1854,5 g TCD-Alkohol DM (Carbonylzahl 0,57 mg KOH/g) mit 94,4 mg Camphersulfonsäure versetzt. Bei der folgenden Destillation wurden 1836,8 g TCD-Alkohol DM mit einer Carbonylzahl von 0,052 mg KOH/g erhalten. Dies entsprach einer destillativen Ausbringung von 98,2%. Die Carbonylzahl wurde auf 9,1% des ursprünglichen Wertes verringert.

### Beispiel 9

Gemäß Beispiel 1 wurden 1968,6 g TCD-Alkohol DM (Carbonylzahl 0,57 mg KOH/g) mit 196,9 mg Camphersulfonsäure versetzt. Bei der folgenden Destillation wurden 1926,3 g TCD-Alkohol DM mit einer Carbonylzahl von 0,074 mg KOH/g erhalten. Dies entsprach einer destillativen Ausbringung von 97,9%. Die Carbonylzahl wurde auf 13,1% des ursprünglichen Wertes verringert.

### Beispiel 10

Gemäß Beispiel 1 wurden 2048,6 g TCD-Alkohol DM (Carbonylzahl 1,17 mg KOH/g) mit 102,4 mg Kaliumhydrogensulfat versetzt. Bei der folgenden Destillation wurden 2025,4 g TCD-Alkohol DM mit einer Carbonylzahl von 0,055 mg KOH/g erhalten. Dies entsprach einer destillativen Ausbringung von 98,9%. Die Carbonylzahl wurde auf 4,7% des ursprünglichen Wertes verringert.

### Beispiel 11

Gemäß Beispiel 1 wurden 1888,1 g TCD-Alkohol DM (Carbonylzahl 1,17 mg KOH/g) mit 150 mg 9-Anthracencarbonsäure versetzt. Bei der folgenden Destillation wurden 1869,5 g TCD-Alkohol DM mit einer Carbonylzahl von 0,69 mg KOH/g erhalten. Dies entsprach einer destillativen Ausbringung von 99,0%. Die Carbonylzahl wurde auf 59,0% des ursprünglichen Wertes verringert.

## Patentansprüche

1. Verfahren zur destillativen Reinigung von alicyclischen Alkoholen, **dadurch gekennzeichnet, dass** man die alicyclischen Alkohole in Gegenwart von 1 bis 500 ppm sauer reagierender Verbindungen, bezogen auf die Menge des eingesetzten alicyclischen Alkohols, destilliert.

2. Verfahren gemäß Anspruch 1, **dadurch gekennzeichnet, dass** die alicyclischen Alkohole 7 bis 17 Kohlenstoffatome im Molekül enthalten.

3. Verfahren nach Anspruch 1 oder 2, **dadurch gekennzeichnet, dass** man als alicyclische Alkohole ein Isomerengemisch enthaltend 3,8-Bis(hydroxymethyl)tricyclo[5.2.1.0^{2,6}]decan, 3,9-Bis(hydroxymethyl)tricyclo[5.2.1.0^{2,6}]decan, 4,8-Bis(hydroxymethyl)tricyclo[5.2.1.0^{2,6}]decan und 4,9-Bis(hydroxymethyl)tricyclo[5.2.1.0^{2,6}]decan, ein Isomerengemisch / enthaltend 8-Hydroxy-3- hydroxymethyltricyclo[5.2.1.0^{2.6}]decan, 9-Hydroxy-3-hydroxymethyltricyclo[5.2.1.0^{2,6}]decan, 8-Hydroxy-4-hydroxymethyltricyclo[5.2.1.0^{2.6}]decan und 9-Hydroxy-4-hydroxymethyltricyclo[5.2.1.0^{2,6}]decan, ein Isomerengemisch enthaltend 1,4:5,8-Dimethano-2,6-bis(hydroxymethyl)perhydrofluoren, 1,4:5,8-Dimethano-2,7-bis(hydroxymethyl)perhydrofluoren, 1,4:5,8-Dimethano-3,6-bis(hydroxymethyl)perhydrofluoren und 1,4:5,8-Dimethano-3,7-bis(hydromethyl)perhydrofluoren und 4,9:5,8-Dimethano-1,6-bis(hydroxymethyl)perhydrobenz[f]inden, 4,9:5,8-Dimethano-2,6-bis(hydroxymethyl)perhydrobenz[f]inden, 4,9:5,8-Dimethano-1,7-bis(hydroxymethyl)perhydrobenz[f]inden und 4,9:5,8-Dimethano-2,7-bis(hydroxymethyl)perhydrobenz[f]inden, oder ein Isomerengemisch enthaltend 2-Hydroxymethylbicyclo[2.2.1]heptan einsetzt.

4. Verfahren nach einem oder mehreren der Ansprüche 1 bis 3, **dadurch gekennzeichnet, dass** man als sauer reagierende Verbindungen aliphatische oder aromatische Sulfonsäuren, aliphatische oder aromatische ein oder mehrwertige Carbonsäuren, saure Salze starker oder schwacher Mineralsäuren, starke oder schwache Mineralsäuren, Tetraalkylammoniumsalze starker oder schwacher Mineralsäuren, feste Nichtmetalloxide oder acide lonenaustauscher verwendet.

5. Verfahren nach einem oder mehreren der Ansprüche 1 bis 4, **dadurch gekennzeichnet, dass** man als sauer reagierende Verbindung para-Toluolsulfonsäure, Naphthalinsulfonsäure, Sulfanilsäure, Camphersulfonsäure, Kaliumhydrogensulfat, Natriumdihydrogenphosphat, Tetrabutylammoniumhydrogensulfat, 9-Anthracencarbonsäure oder Diphosphorpentoxid verwendet.

6. Verfahren nach einem oder mehreren der Ansprüche 1 bis 5, **dadurch gekennzeichnet, dass** man die sauer reagierenden Verbindungen in einer Menge von 5-250 ppm, vorzugsweise in einer Menge von 5-50 ppm, jeweils bezogen auf die Menge des alicyclischen Alkohols, zusetzt.

7. Verfahren nach einem oder mehreren der Ansprüche 1 bis 6, **dadurch gekennzeichnet, dass** man die Destillation bei einer Temperatur von 100 bis 240°C durchführt.

## Claims

1. A process for purifying alicyclic alcohols by distillation, which comprises distilling the alicyclic alcohols in the presence of from 1 to 500 ppm of acidic compounds, based on the amount of the alicyclic alcohol used.

2. The process as claimed in claim 1, wherein the alicyclic alcohols have from 7 to 17 carbon atoms in the molecule.

3. The process as claimed in claim 1 or 2, wherein the alicyclic alcohol used is an isomer mixture comprising 3,8-bis(hydroxymethyl)tricyclo-[5.2.1.0^{2,6}]decane, 3,9-bis(hydroxymethyl)tricyclo[5.2.1.0^{2,6}]decane, 4,8-bis(hydroxymethyl)tricyclo[5.2.1.0^{2,6}]decane) and 4,9-bis-(hydroxymethyl)tricyclo[5.2.1.0^{2,6}]decane, an isomer mixture comprising 8-hydroxy-3-hydroxymethyltricyclo[5.2.1.0^{2,6}]decane, 9-hydroxy-3-hydroxymethyltricyclo[5.2.1.0^{2,6}]decane, 8-hydroxy-4-hydroxymethyltricyclo[5.2.1.0^{2,6}]decane and 9-hydroxy-4-hydroxymethyltricyclo[5.2.1.0^{2,6}]decane, an isomer mixture comprising 1,4:5,8-dimethano-2,6-bis(hydroxymethyl)perhydrofluorene, 1,4:5,8-dimethano-2,7-bis(hydroxymethyl)perhydrofluorene, 1,4:5,8-dimethano-3,6-bis(hydroxymethyl)perhydrofluorene and 1,4:5,8-dimethano-3,7-bis(hydromethyl)perhydrofluorene, and
4,9:5,8-dimethano-1,6-bis(hydroxymethyl)perhydrobenz[f]indene, 4,9:5,8-dimethano-2,6-bis(hydroxymethyl)perhydrobenz[f]indene, 4,9:5,8-dimethano-1,7-bis(hydroxymethyl)perhydrobenz[f]indene and 4,9:5,8-dimethano-2,7-bis(hydroxymethyl)perhydrobenz[f]indene or an isomer mixture comprising 2-hydroxymethylbicyclo[2.2.1]heptane.

4. The process as claimed in one or more of claims 1 to 3, wherein acidic compounds used are aliphatic or aromatic sulfonic acids, aliphatic or aromatic monobasic or polybasic carboxylic acids, acid salts of strong or weak mineral acids, strong or weak mineral acids, tetraalkylammonium salts of strong or weak mineral acids, solid nonmetal oxides or acidic ion exchangers.

5. The process as claimed in one or more of claims 1 to 4, wherein the acidic compound used is para-toluenesulfonic acid, naphthalenesulfonic acid, sulfanilic acid, camphorsulfonic acid, potassium hydrogen sulfate, sodium dihydrogen phosphate, tetrabutylammonium hydrogen sulfate, 9-anthracenecarboxylic acid or diphosphorus pentoxide.

6. The process as claimed in one or more of claims 1 to 5, wherein the acidic compounds are added in an amount of 5-250 ppm, preferably in an amount of 5-50 ppm, in each case based on the amount of the alicyclic alcohol.

7. The process as claimed in one or more of claims 1 to 6, wherein the distillation is carried out at a temperature of from 100 to 240°C.

## Revendications

1. Procédé pour la purification par distillation d'alcools alicycliques, **caractérisé en ce qu'**on distille les alcools alicycliques en présence de 1 à 500 ppm de composés à réaction acide, par rapport à la quantité d'alcool alicyclique utilisé.

2. Procédé selon la revendication 1, **caractérisé en ce que** les alcools alicycliques contiennent 7 à 17 atomes de carbone dans la molécule.

3. Procédé selon la revendication 1 ou 2,
**caractérisé en ce qu'**on utilise comme alcools alicycliques un mélange d'isomères contenant du 3,8-bis(hydroxyméthyl)tricyclo[5.2.1.0^{2,6}]décane, du 3,9-bis(hydroxyméthyl)tricyclo[5.2.1.0^{2,6}]décane, du 4,8-bis(hydroxyméthyl)tricyclo[5.2.1.0^{2,6}]décane et du 4,9-bis(hydroxyméthyl)tricyclo[5.2.1.0^{2,6}]décane, un mélange d'isomères contenant du 8-hydroxy-3-hydroxyméthyltricyclo[5.2.1.0^{2,6}]décane, du 9-hydroxy-3-hydroxyméthyltricyclo[5.2.1.0^{2,6}]décane, du 8-hydroxy-4-hydroxyméthyltricyclo[5.2.1.0^{2,6}]décane et du 9-hydroxy-4-hydroxyméthyltricyclo[5.2.1.0^{2,6}]décane, un mélange d'isomères contenant du 1,4:5,8-diméthano-2,6-bis(hydroxyméthyl)perhydrofluorène, du 1,4:5,8-diméthano-2,7-bis(hydroxyméthyl)perhydrofluorène, du 1,4:5,8-diméthano-3,6-bis(hydroxyméthyl)perhydrofluorène et du 1,4:5,8-diméthano-3,7-bis-(hydrométhyl)perhydrofluorène et du 4,9:5,8-diméthano-1,6-bis(hydroxyméthyl)perhydrobenz[f]indène, du 4,9:5,8-diméthano-2,6-bis(hydroxyméthyl)perhydrobenz-[f]indène, du 4,9:5,8-diméthano-1,7-bis(hydroxyméthyl)-perhydrobenz[f]indène et du 4,9:5,8-diméthano-2,7-bis-(hydroxyméthyl)perhydrobenz[f]indène, ou un mélange d'isomères contenant du 2-hydroxyméthylbicyclo[2.2.1]heptane.

4. Procédé selon l'une ou plusieurs des revendications 1 à 3, **caractérisé en ce qu'**on utilise comme composés à réaction acide des acides sulfoniques aliphatiques ou aromatiques, des acides carboxyliques aliphatiques ou aromatiques monovalents ou polyvalents, des sels acides d'acides minéraux forts ou faibles, des acides minéraux forts ou faibles, des sels de tétraalkylammonium d'acides forts ou faibles, des oxydes non métalliques solides ou des échangeurs d'ions acides.

5. Procédé selon l'une ou plusieurs des revendications 1 à 4, **caractérisé en ce qu'**on utilise comme composé à réaction acide l'acide para-toluènesulfonique, l'acide naphtalènesulfonique, l'acide sulfanilique, l'acide camphresulfonique, l'hydrogénosulfate de potassium, le dihydrogénophosphate de sodium, l'hydrogénosulfate de tétrabutylammonium, l'acide 9-anthracènecarboxylique ou le pentoxyde diphosphorique.

6. Procédé selon l'une ou plusieurs des revendications 1 à 5, **caractérisé en ce qu'**on ajoute les composés à réaction acide en une quantité de 5-250 ppm, de préférence en une quantité de 5-50 ppm, à chaque fois par rapport à la quantité de l'alcool alicyclique.

7. Procédé selon l'une ou plusieurs des revendications 1 à 6, **caractérisé en ce qu'**on réalise la distillation à une température de 100 à 240°C.
